# EUROPEAN PATENT SPECIFICATION

(11) **EP 2 083 699 B1**
(45) Date of publication and mention of the grant of the patent: **19.04.2017**
(21) Application number: 07798690.9
(22) Date of filing: 18.06.2007
(51) Int. Cl.: A61B 10/06, A61B 18/14, A61B 10/02, A61B 10/04, A61B 18/22

(54) **TISSUE SAMPLE PROTECTING CAUTERIZING BIOPSY FORCEPS**
GEWEBESCHÜTZENDE KAUTERISIERENDE BIOPSIEZANGE
FORCEPS DE BIOPSIE ET DE CAUTÉRISATION PROTÉGEANT UN ÉCHANTILLON DE TISSUS

(30) Priority: 12.09.2006 US 843963 P; 15.06.2007 US 763586
(43) Date of publication of application: 05.08.2009
(73) Proprietor: Boston Scientific Scimed, Inc., Maple Grove, MN 55311-1566 (US)
(72) Inventor: NOWLIN, Brett, Bridgewater, MA 02324 (US); DAIGNAULT, Ken, Holden, MA 01520 (US); CHU, Michael S.H., Brookline, MA 02446 (US)
(74) Representative: Vossius & Partner Patentanwälte Rechtsanwälte mbB
(86) International application number: PCT/US2007/071444
(87) International publication number: WO 2008/033593

(56) References cited:
- WO-A-2007/040800
- US-A- 5 226 908
- US-A- 5 295 990
- US-A- 5 743 906
- US-A1- 2002 049 442
- US-A1- 2003 055 423
- US-A1- 2005 085 808

## Description

### Background Information

Biopsy is the preferred method of diagnosis for many diseases and conditions. When it is desired to biopsy tissue within certain body structures such as, for example, hollow organs or lumens, an endoscope or similar device may be used to reach target issue and to insert cutting and retrieval devices to that tissue via the working lumen of the scope.

Because of the cutting involved, cauterization elements are often inserted to the sampling site to stop the bleeding that may occur at the sampling site. These cauterization elements may be part of a separate device or may be included in the cutting and/or retrieval devices used to obtain the biopsy. However, it may be difficult with conventional devices to achieve a desired degree of cauterization (i.e., sufficient to stop the bleeding) without excessive damage to surrounding tissue. In addition, exposure to excessive heat may damage the tissue sample reducing or destroying its diagnostic value.

US5743906 discloses a biopsy forceps with cauterization electrodes.

The present invention is defined in appended claim 1, preferred embodiments are described in the dependent claims.

The present invention relates to a biopsy device which includes a tissue cutting arrangement and a cauterizing element. The tissue cutting arrangement is operable to excise a tissue sample from a sampling site. The tissue cutting arrangement defines a sample chamber therewithin for receiving a tissue sample after excision by the tissue cutting arrangement. The sample chamber includes insulated sample contacting surfaces. The cauterizing element is formed on at least a portion of an exterior surface of the tissue cutting arrangement. The cauterizing element is actuatable to cauterize the sampling site after excision of the tissue sample.

Also disclosed is a method of biopsy. An insulated biopsy forceps is advanced to a sampling site within a body lumen. A tissue sample from the sampling site is actuated from a cutting element of the forceps from outside the body to excise. The tissue sample is retained in a sample chamber within the forceps, the sample chamber having insulated interior surfaces. A cauterizing element is energized to cauterize the sampling site.

The present invention further relates to a biopsy system with cauterization. The system may include a shaft having a distal end insertable through a scope to a sampling site within a body; a non-conductive shaft coating covering at least a portion of the shaft; a housing disposed at a distal end of the shaft; a cutting element of the housing operable by a user to excise a sample tissue from the sampling site; a sample chamber defined within the housing for retaining the excised sample tissue; a cauterization element of the housing to cauterize the sampling site after excision of the sample tissue; and a non-conductive housing coating covering at least parts of surfaces of the housing.

### Brief Description of Drawings

Figure 1 is a perspective view showing an arrangement of a biopsy forceps not according to the invention in an open configuration;
Figure 2 is perspective view of the biopsy forceps shown in Fig. 1 in the closed configuration;
Figure 3 is a view of the biopsy forceps shown in Fig. 1 attached to a carrier device;
Figure 4 is a perspective view of a biopsy forceps according to an embodiment of the present invention, in a closed configuration;
Figure 5 is a perspective view of the biopsy forceps shown in Fig. 4, in an open configuration;
Figure 6 is a perspective view of a biopsy forceps according to an arrangement not according to the present invention, with a separate cauterization element;
Figure 7 is a perspective view of a biopsy forceps according to a different arrangement not according to the present invention, with a laser cauterization element; and
Figure 8 is a perspective view of a biopsy forceps according another arrangement not according to the present invention, comprising a sample basket.

### Detailed Description

The present invention may be further understood with reference to the following description and the appended drawings, wherein like elements are referred to with the same reference numerals. The present invention relates to devices for performing a biopsy. More specifically, the invention relates to devices used to retrieve biopsy tissue samples and to cauterize the resulting wound.

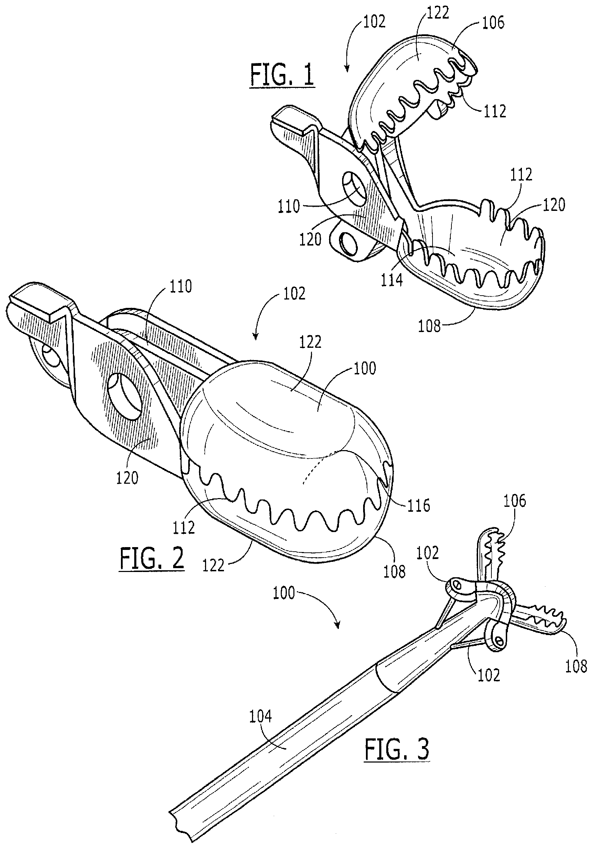

As shown in Fig. 1, a biopsy forceps 100 according to the present invention adapted to obtain a sample of tissue and immediately cauterize the sample site while preserving the tissue sampled therefrom comprises a shaft 104 used to advance through, for example, an endoscope the distal head 102 to the target sample area. The distal head 102 comprises the cutting instruments used to remove the sample tissue from its surroundings.

The forceps 100 according to an exemplary arrangement comprises a pair of hinged jaws 106, 108 including overlapping teeth 112, or other similar cutting surfaces. The opposing cutting surfaces of the jaws 106, 108 may be brought together over the sample tissue once the distal head 102 has been advanced to the sampling site within, for example, a body lumen or cavity. The exemplary jaws 106, 108 pivot about a hinge 110, so that they can open and close using a remote actuator 104, that may be operated by a user from outside the patient's body.

The jaws 106, 108 are preferably shaped to form a sample holder, such as a sample cup 114 that becomes an enclosed sample chamber 116 once the jaws 106, 108 are closed. The sample chamber 116 retains the sample for removal from the body while protecting the sample from contamination during removal. The jaws 106, 108 are preferably designed to automatically enclose the sample within the chamber 116 after the sample has been excised to prevent loss of the sample during retrieval. Those of skill in the art will understand that different shapes and locations of the sample chamber 116 may be used, without departing the scope of the present invention.

Multiple surfaces of the biopsy forceps 100 are coated with an insulating material to prevent the non target tissue and the sample tissue from being damaged. For example, the insulation may comprise PTFE or PFA materials, ceramics or any other coating that reduces heat transfer and is electrically non-conductive. The insulating coating may be sprayed, dipped or painted on the device. As shown in the drawings, the device according to this embodiment comprises coated, non-conductive surfaces 120 formed as described above as well as non-coated, conductive surfaces 122 which deliver energy to cauterize the sampling site. A large portion of the exemplary device is coated with the non-conductive insulation, including the shaft 104 and the sample cup 114. As would be understood by those skilled in the art, for forceps according to alternate embodiments using other forms of energy (e.g., laser) to cauterize tissue, the insulation need not be electrically non-conductive and may be simply thermally insulative. For example, an electrically conductive, thermally insulative material such as conductive acetal polymer black carbon filled Pomaflux CN-F may be used to the shaft 104 and/or the sample cup 114. A laser may be used to heat the jaws 106, 108 for cauterizing tissue when need to be thermally insulated but not electrically insulated.

When the jaws 106, 108 are closed, the conductive portions 122 form a generally bullet shaped cauterization tool that may be used to stop bleeding within the sampling site. The user may, for example, visualize the site via an endoscope to direct the distal head 102 to the site of bleeding to cauterize and seal the wound. Because of the insulation disposed on the surfaces 120, the portion of tissue cauterized may be accurately targeted minimizing inadvertent cauterization of the surrounding tissue. The sample within the sample chamber 116 is also protected from damage during the cauterization steps by insulation coated on the inside portions of the jaws 106, 108.

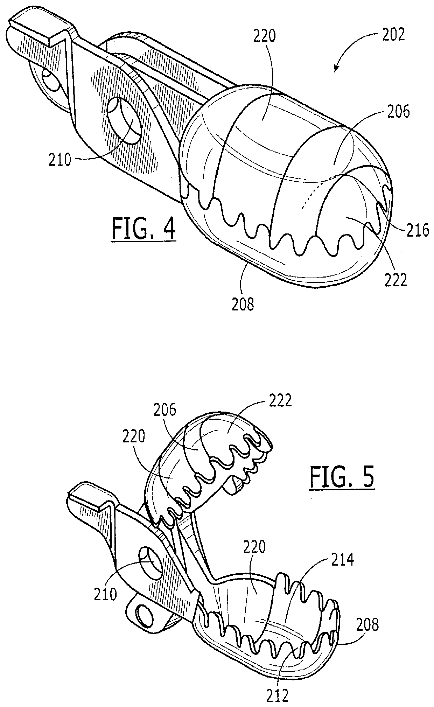

An embodiment of a biopsy forceps according to the present invention is shown in Figs. 4 and 5. In this embodiment the conductive region of the device is smaller and shaped to more closely direct RF energy path in a selected direction. As shown, the forceps 202 comprises a pair of jaws 206, 208 having a cutting portion 212 for removing tissue samples. A cup 214 is preferably formed by the jaws 206, 208 to hold the tissue sample during retrieval and large portions of the jaws 206, 208 are coated with a non-conductive material to form non-conductive surfaces 220 that extend over the internal portions of the jaws 206, 208 to protect the sample tissue.

In this embodiment, the conductive surfaces 222 of the device are limited to the distal tip of the jaws 206, 208. For example, the outer distal ends of the forceps 202 are left uncoated to form a substantially circular cauterization zone. According to this embodiment, the shape of the conductive surfaces 222 directs RF energy toward a grounding pad and away from the sample of tissue contained within the sample chamber 216. In other exemplary arrangements, the conductive surfaces 222 may be located on external surfaces of sidewalls of the jaws 206, 208, e.g., about 180° apart. For example, the conductive surfaces 222 may be located on opposite sides of the jaws 206, 208 adjacent the contacting edges and/or on upper external portion of the jaw 206 and a lower external portion of the jaw 208 when the contacting edges are in a horizontal plane. In this manner, the forceps 202 need only be rotated, at most, about 90° to align one of the conductive surfaces 222 with the target tissue region to be cauterized. In another exemplary embodiment, the conducting surfaces 222 may be selectively activated, e.g., one on/one off. Preferably, the conductive surfaces 222 are semi-circular, forming a substantially circular shape when the jaws 206, 208 are closed.

The preceding embodiments of the biopsy forceps may be based on existing forceps used for sample tissue retrieval. For example, the Barracuda and the Tiger Shark lines of biopsy forceps, and the Stone Retrieval Devices Basket line manufactured by the Boston Scientific Corporation of Natick, MA, may be modified to operate in accord with the teachings of the present invention by applying non-conductive coatings in to selected areas as described above.

In alternate arrangements, the jaws or cutting elements of the device are not used to perform the cauterization of the sampling area. Instead, a separate media is used to cauterize the wound, for example by directing RF or other energy to the target tissue. According to these arrangements, a path of least resistance is provided toward the tissue to be cauterized (e.g., toward a grounding pad located on an opposite side of the tissue to be cauterized) and away from the sample tissue held within the device. The cauterization media may be a fixed or a movable protrusion that extends from the biopsy forceps towards the target tissue.

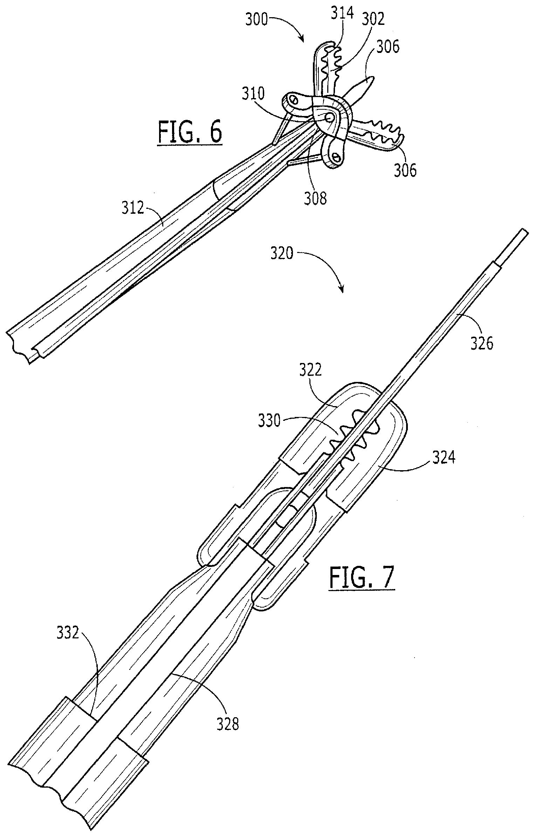

As shown in Fig. 6, forceps 300 according to an exemplary arrangement comprises a pair of jaws 302, 304 at the distal end of a shaft 312 of the forceps 300 forming a sample chamber 314 when pivoted about the hinge 310 to the closed configuration. The forceps 300 may further include a tissue grabber 306 which telescopes distally from a lumen 308 of the shaft 312 to hold tissue in place. The forceps 300 further includes an optic fiber 326 (shown in Fig. 7) which, during insertion of the device and excision of the tissue, is preferably stored within the shaft 312 and, after the sample has been placed within the sample chamber 314, extended to the site from which the tissue was removed to cauterize the resulting wound. Alternatively, the optic fiber 326 may be fixed in an extended position and activated only after the tissue sample has been enclosed within the sample chamber 314.

A biopsy forceps 320 as shown in Fig. 7, comprises a pair of pivoting jaws 322, 324 that include cutting elements to remove the sample tissue from the sampling site. As described above in regard to the previous embodiments, the jaws 322, 324 close to form a sample chamber 330 in which the tissue sample is stored for removal from the body. The laser light is transmitted to the distal end of the forceps 320 via the optic fiber 326 to a selected position for irradiating tissue in a desired spatial relation to the distal end of the forceps 320. As would be understood by those skilled in the art, heat transferred to the sample within the sample chamber 330 as the laser energy passes by via the optic fiber is minimal and only a small amount of heat is transferred to non-targeted surrounding tissue on which the laser is not aimed.

Furthermore as would be understood by those skilled in the art, other mechanisms and/or forms of energy may be used to cauterize the tissue. For example, a resistance type heater may be utilized instead of the laser and RF energy systems described above. Alternatively, a dual lumen may be provided in the shaft of the biopsy forceps according to the invention through which a wire loop (e.g., of nickel-chrome wire) for RF or other forms of electric energy may be advanced to the sampling site to cauterize the tissue. As would be understood, such devices may be powered by an external battery, RF generator or other power source and, as in the embodiments described above, the loops may be fixed or may be telescoping from the shaft of the device.

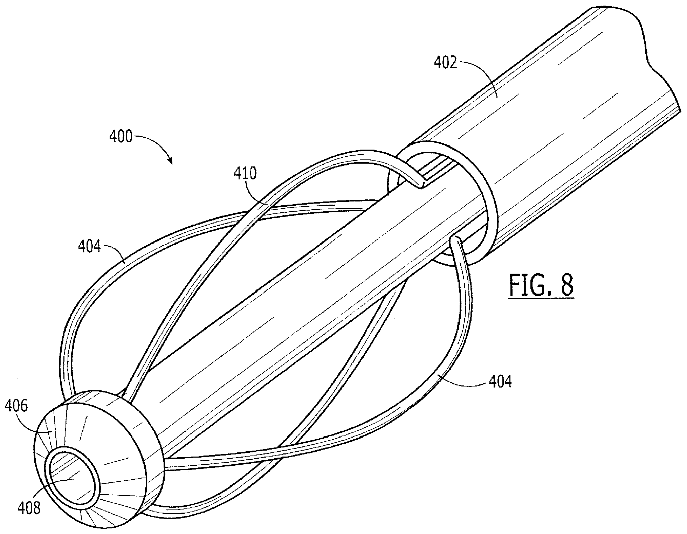

As shown in Fig. 8, a biopsy forceps 400 according to a further arrangement includes a basket 410 which captures the tissue sample after it has been excised. The basket 410 of the forceps 400 comprises a channel 408 opening at the distal end 406 and accommodating a heating element such as an RF wire, a fiber-optic cable for delivering laser energy, a nickel-chrome wire or other device which, when passed through the channel 408 to a location adjacent to the target site, may be energized to cauterize the tissue surrounding the site from which the tissue sample was removed. As described above, for electric and RF cauterizing systems, outer surfaces of the channel 408 are preferably made non-conductive, and may be used as an insulator. Teflon PTFE, Teflon PFA , ceramic or other heat resistant materials may be used to form the coating. Of course, such coatings need not be electrically non-conductive for laser based systems.

In another arrangement, the basket 410 is formed of a plurality of filaments 404 with one or more of the filaments 404 transmitting energy to the target tissue. For example, the one or more filaments 404 may be formed of an electrically conductive wire or fiber-optic cable to transmit RF, laser or other energy to a cauterization element disposed at the distal tip 406. For example, where one of the filaments 404 is used as a hot element for cauterization, the location of this filament 404 with respect to the interior of the basket 410 is selected to minimize the affect of the heat on a tissue sample retained within the basket 410. In addition, selected portions of the filament 404 may be insulated (thermally and/or electrically) to maximize energy transfer to the target tissue while minimizing energy transfer to sample tissue retained within the basket 410.

The present invention has been described with reference to specific exemplary embodiments. Those skilled in the art will understand that changes may be made in details, particularly in matters of shape, size, material and arrangement of parts. Accordingly, various modifications and changes may be made to the embodiments. The specifications and drawings are, therefore, to be regarded in an illustrative rather than a restrictive sense.

## Claims

1. A biopsy device (100, 200), comprising:
a tissue cutting arrangement operable to excise a tissue sample from a sampling site, the tissue cutting arrangement including a housing (102, 202, 300, 400) defining a sample chamber (116, 216, 314) therewithin for receiving a tissue sample after excision by the tissue cutting arrangement, the sample chamber (116, 216, 314) including insulated sample contacting surfaces; and a cauterizing element formed on an exterior surface of the housing (102,202,300,400), the cauterizing element being actuatable to cauterize the sampling site after excision of the tissue sample, **characterized in that**
the cauterizing element is limited to a distal tip of an exterior surface of the housing (102,202,300,400). a cauterizing element limited to a distal tip of a surface of the housing (102, 202, 300, 400).

2. The biopsy device of claim 1, wherein at least a portion of the housing (102, 202, 300, 400) is formed of an electrically conductive material, a first portion (122, 222) of the surface of the housing (102, 202, 300, 400) forming the tissue cauterizing element being substantially free of an electrically insulating material and a second portion (220) of the surface of the housing (102, 202, 300, 400) not forming the tissue cauterizing element being covered in an electrically insulative material.

3. The biopsy device of claim 1, wherein the housing (102, 202, 300, 400) comprises hinged jaws (106, 108, 206, 208, 302, 306, 322, 324), inner surfaces of the hinged jaws forming the sample chamber being coated with an insulating material.

4. The biopsy device of claim 3, wherein, when the hinged jaws (106, 108, 206, 208, 302, 306, 322, 324) are in a closed configuration, selected uninsulated portions of outer surfaces of the hinged jaws form a substantially circular electrically conductive region.

5. The biopsy device of claim 1, wherein the insulated sample contacting surfaces (120, 220) are coated with one of PTFE, PFA and ceramic.

6. The biopsy device of claim 1, wherein the insulating sample contacting surfaces are electrically insulated.

7. The biopsy device of claim 1, further comprising: an electrically conductive element extending proximally from the cauterizing element to a source of electric energy, the conductive element telescoping from a lumen of the tissue cutting arrangement, the lumen insulating an interior of the sample chamber from the conductive element.

8. The biopsy device of claim 1, wherein the cauterizing element comprises an optic fiber (326) connected to a laser, the optic fiber telescoping from a lumen of the tissue cutting arrangement.

9. The biopsy device of claim 1, wherein the sample chamber (116, 216, 314) comprises a basket (410) formed of a plurality of filaments (404).

10. A biopsy system with cauterization, comprising a biopsy device according to claim 1, wherein the tissue cutting arrangement comprises
a first shaft (104, 312) having a distal end insertable through a scope to a sampling site within a body;
a second non-conductive shaft coating covering at,least a portion of the first shaft (104, 312);
a the housing (102, 202, 300, 400) disposed at a distal end of the first shaft (104, 312);
a cutting element of the housing (102, 202, 300, 400) operable by a user to excise a sample tissue from the sampling site;
and wherein the housing (102, 202, 300, 400) is covered at least in parts of the surface of the housing (102, 202, 300, 400) with a non-conductive coating.

11. The biopsy system of claim 10, wherein the sample chamber is formed by non-conductive forceps cups (114) defined within a pair of jaws (106, 108, 206, 208, 302, 306, 322, 324).

12. The biopsy system of claim 10, wherein the cauterization element comprises uninsulated portions (122, 222) of a pair of pivotally connected jaws (106, 108, 206, 208, 302, 306, 322, 324).

13. The biopsy system of claim 10, wherein the non-coated portion is substantially circular when the jaws (106, 108, 206, 208, 302, 306, 322, 324) are closed.

14. The biopsy system of claim 10, wherein the cauterization element comprises a source of laser energy coupled to an optic fiber (326) extendable distally from the housing (102, 202, 300, 400).

15. The biopsy system of claim 10, wherein the shaft coating and the housing coating comprise at least one of PTFE, PFA and ceramic material.

## Patentansprüche

1. Biopsievorrichtung (100, 200), mit:
einer Gewebeschneidanordnung, die betätigbar ist, eine Gewebeprobe aus einer Probenahmestelle zu exzidieren, wobei die Gewebeschneidanordnung ein Gehäuse (102, 202, 300, 400) aufweist, das darin eine Probenkammer (116, 216, 314) zum Aufnehmen einer Gewebeprobe nach der Exzision durch die Gewebeschneidanordnung definiert, wobei die Probenkammer (116, 216, 314) isolierte Probenkontaktflächen aufweist; und
ein Kauterisationselement, das an einer Außenfläche des Gehäuses (102, 202, 300, 400) ausgebildet ist, wobei das Kauterisationselement betätigbar ist, die Probenahmestelle nach der Exzision der Gewebeprobe zu kauterisieren, **dadurch gekennzeichnet, dass** das Kauterisationselement auf eine distale Spitze einer Außenfläche des Gehäuses (102, 202, 300, 400) begrenzt ist.

2. Biopsievorrichtung nach Anspruch 1, wobei mindestens ein Abschnitt des Gehäuses (102, 202, 300, 400) aus einem elektrisch leitfähigen Material ausgebildet ist, wobei ein erster Abschnitt (122, 222) der Oberfläche des Gehäuses (102, 202, 300, 400), der das Gewebekauterisationselement bildet, im Wesentlichen von einem elektrisch isolierenden Material frei ist und ein zweiter Abschnitt (220) der Oberfläche des Gehäuses (102, 202, 300, 400), der das Gewebekauterisationselement nicht bildet, in ein elektrisch isolierendes Material eingehüllt ist.

3. Biopsievorrichtung nach Anspruch 1, wobei das Gehäuse (102, 202, 300, 400) drehbar angebrachte Backen (106, 108, 206, 208, 302, 306, 322, 324) aufweist, wobei Innenflächen der drehbar angebrachten Backen die Probenkammer bilden, die mit einem isolierenden Material überzogen ist.

4. Biopsievorrichtung nach Anspruch 3, wobei dann, wenn sich die drehbar angebrachten Backen (106, 108, 206, 208, 302, 306, 322, 324) in einer geschlossenen Konfiguration befinden, ausgewählte unisolierte Abschnitte der Außenflächen der drehbar angebrachten Backen einen im Wesentlichen kreisförmigen elektrisch leitfähigen Bereich bilden.

5. Biopsievorrichtung nach Anspruch 1, wobei die isolierten Probenkontaktflächen (120, 220) mit PTFE oder PFA oder Keramik beschichtet sind.

6. Biopsievorrichtung nach Anspruch 1, wobei die isolierenden Probenkontaktflächen elektrisch isoliert sind.

7. Biopsievorrichtung nach Anspruch 1, die ferner aufweist: ein elektrisch leitfähiges Element, das sich proximal vom Kauterisationselement zu einer Quelle elektrischer Energie erstreckt, wobei sich das leitfähige Element zusammenschiebbar von einem Lumen der Gewebeschneidanordnung erstreckt, wobei das Lumen ein Inneres der Probenkammer vom leitfähigen Element isoliert.

8. Biopsievorrichtung nach Anspruch 1, wobei das Kauterisationselement eine optische Faser (326) aufweist, die mit einem Laser verbunden ist, wobei sich die optische Faser zusammenschiebbar von einem Lumen der Gewebeschneidanordnung erstreckt.

9. Biopsievorrichtung nach Anspruch 1, wobei die Probenkammer (116, 216, 314) einen Korb (410) aufweist, der aus mehreren Filamenten (404) ausgebildet ist.

10. Biopsiesystem mit Kauterisation, das eine Biopsievorrichtung nach Anspruch 1 aufweist, wobei die Gewebeschneidanordnung aufweist:
einen ersten Schaft (104, 312), der ein distales Ende aufweist, das durch ein Skop zu einer Probenahmestelle innerhalb eines Körpers einführbar ist;
eine zweite nicht leitfähige Schaftbeschichtung, die mindestens einen Abschnitt des ersten Schafts (104, 312) bedeckt;
wobei das Gehäuse (102, 202, 300, 400) an einem distalen Ende des ersten Schafts (104, 312) angeordnet ist;
wobei ein Schneidelement des Gehäuses (102, 202, 300, 400), durch einen Benutzer betätigbar ist, um eine Gewebeprobe von der Probenahmestelle zu exzidieren;
und wobei das Gehäuse (102, 202, 300, 400) mindestens in Teilen der Oberfläche des Gehäuses (102, 202, 300, 400) mit einem nicht leitfähigen Überzug bedeckt ist.

11. Biopsiesystem nach Anspruch 10, wobei die Probenkammer durch nicht leitfähige Zangenschalen (114) gebildet wird, die in einem Backenpaar (106, 108, 206, 208, 302, 306, 322, 324) definiert sind.

12. Biopsiesystem nach Anspruch 10, wobei das Kauterisationselement unisolierte Abschnitte (122, 222) eines Paars schwenkbar verbundener Backen (106, 108, 206, 208, 302, 306, 322, 324) aufweist.

13. Biopsiesystem nach Anspruch 10, wobei der nicht überzogene Abschnitt im Wesentlichen kreisförmig ist, wenn die Backen (106, 108, 206, 208, 302, 306, 322, 324) geschlossen sind.

14. Biopsiesystem nach Anspruch 10, wobei das Kauterisationselement eine Quelle von Laserenergie aufweist, die mit einer optischen Faser (326) gekoppelt ist, die distal vom Gehäuse (102, 202, 300, 400) ausfahrbar ist.

15. Biopsiesystem nach Anspruch 10, wobei die Schaftbeschichtung und die Gehäusebeschichtung PTFE und/oder PFA und/oder ein Keramikmaterial aufweisen.

## Revendications

1. Dispositif de biopsie (100, 200), comprenant :
un système de découpe de tissus permettant d'exciser un échantillon de tissu d'un site d'échantillonnage, le système de découpe de tissus comportant un boîtier (102, 202, 300, 400) définissant une chambre à échantillon (116, 216, 314) intérieure pour la réception d'un échantillon de tissu après excision par le système de découpe de tissus, la chambre à échantillon (116, 216, 314) présentant des surfaces de contact d'échantillon isolées ;
et
un élément de cautérisation formé sur une surface extérieure du boîtier (102, 202, 300, 400), l'élément de cautérisation permettant de cautériser le site d'échantillonnage après excision de l'échantillon de tissu, **caractérisé en ce que**
l'élément de cautérisation est limité à une extrémité distale d'une surface extérieure du boîtier (102, 202, 300, 400).

2. Dispositif de biopsie selon la revendication 1, où au moins une partie du boîtier (102, 202, 300, 400) est constituée d'un matériau électriquement conducteur, une première partie (122, 222) de la surface du boîtier (102, 202, 300, 400) formant l'élément de cautérisation de tissu étant pratiquement exempte de matériau électriquement isolant et une deuxième partie (220) de la surface du boîtier (102, 202, 300, 400) ne formant pas l'élément de cautérisation de tissu étant revêtue d'un matériau électriquement isolant.

3. Dispositif de biopsie selon la revendication 1, où le boîtier (102, 202, 300, 400) comprend des mâchoires articulées (106, 108, 206, 208, 302, 306, 322, 324), les surfaces intérieures des mâchoires articulées formant la chambre à échantillon étant revêtues d'un matériau isolant.

4. Dispositif de biopsie selon la revendication 3, où, dans une configuration de fermeture des mâchoires articulées (106, 108, 206, 208, 302, 306, 322, 324), des parties non isolées sélectionnées de surfaces extérieures des mâchoires articulées forment une zone électriquement conductrice sensiblement circulaire.

5. Dispositif de biopsie selon la revendication 1, où les surfaces de contact d'échantillon isolées (120, 220) sont revêtues soit de PTFE, soit de PFA, soit de céramique.

6. Dispositif de biopsie selon la revendication 1, où les surfaces isolantes de contact d'échantillon sont électriquement isolées.

7. Dispositif de biopsie selon la revendication 1, comprenant en outre : un élément électriquement conducteur s'étendant dans la direction proximale depuis l'élément de cautérisation vers une source de courant électrique, l'élément conducteur s'étendant télescopiquement depuis une lumière du système de découpe de tissus, la lumière isolant l'intérieur de la chambre à échantillon de l'élément conducteur.

8. Dispositif de biopsie selon la revendication 1, où l'élément de cautérisation comprend une fibre optique (326) reliée à un laser, la fibre optique s'étendant télescopiquement depuis une lumière du système de découpe de tissus.

9. Dispositif de biopsie selon la revendication 1, où la chambre à échantillon (116, 216, 314) comprend une corbeille (410) constituée d'une pluralité de filaments (404).

10. Système de biopsie à cautérisation, comprenant un dispositif de biopsie selon la revendication 1, où le système de découpe de tissus comprend
une première tige (104, 312) présentent une extrémité distale insérable dans un endoscope vers un site d'échantillonnage à l'intérieur du corps ;
un deuxième revêtement de tige non conducteur couvrant au moins une partie de la première tige (104, 312) ;
le boîtier (102, 202, 300, 400) disposé à une extrémité distale de la première tige (104, 312) ;
un élément de découpe du boîtier (102, 202, 300, 400) permettant à un utilisateur d'exciser un échantillon de tissu du site d'échantillonnage ;
et où le boîtier (102, 202, 300, 400) est revêtu d'un revêtement non conducteur au moins sur des parties de la surface du boîtier (102, 202, 300, 400).

11. Système de biopsie selon la revendication 10, où la chambre à échantillon est formée par des coupelles de forceps (114) non conductrices définies à l'intérieur d'une paire de mâchoires (106, 108, 206, 208, 302, 306, 322, 324).

12. Système de biopsie selon la revendication 10, où l'élément de cautérisation présente des parties non isolées (122, 222) d'une paire de mâchoires (106, 108, 206, 208, 302, 306, 322, 324) pivotantes articulées.

13. Système de biopsie selon la revendication 10, où la partie non revêtue est sensiblement circulaire quand les mâchoires (106, 108, 206, 208, 302, 306, 322, 324) sont fermées.

14. Système de biopsie selon la revendication 10, où l'élément de cautérisation comprend une source d'énergie laser reliée à une fibre optique (326) extensible dans la direction distale depuis le boîtier (102, 202, 300, 400).

15. Système de biopsie selon la revendication 10, où le revêtement de tige et le revêtement de boîtier comprennent un matériau PTFE et/ou un matériau PFA et/ou un matériau céramique.
